(19) Europäisches Patentamt
European Patent Office
Office européen des brevets

(11) Veröffentlichungsnummer : **0 034 746**
**B1**

(12) # EUROPÄISCHE PATENTSCHRIFT

(45) Veröffentlichungstag der Patentschrift : **18.01.84**

(51) Int. Cl.³ : **C 07 D307/52**, C 07 D333/20, C 07 C147/12, A 61 K 31/63

(21) Anmeldenummer : **81100911.7**

(22) Anmeldetag : **10.02.81**

(54) **5-Sulfamoyl-orthanilsäuren, Verfahren zu ihrer Herstellung, diese enthaltende Heilmittel und die Verbindungen zur Verwendung als Heilmittel.**

(30) Priorität : **22.02.80 DE 3006686**

(43) Veröffentlichungstag der Anmeldung : **02.09.81 Patentblatt 81/35**

(45) Bekanntmachung des Hinweises auf die Patenterteilung : **18.01.84 Patentblatt 84/03**

(84) Benannte Vertragsstaaten : **AT BE CH DE FR GB IT LI NL SE**

(56) Entgegenhaltungen :
**AT-B- 315 827**
**DE-A- 2 142 758**
**DE-A- 2 232 457**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Sturm, Karl, Dr.**
**Berndesallee 64**
**D-6501 Heidesheim (DE)**
Erfinder : **Muschaweck, Roman, Dr.**
**Heimchenweg 39**
**D-6230 Frankfurt am Main 80 (DE)**

**EP 0 034 746 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

5-Sulfamoyl-orthanilsäuren, Verfahren zu ihrer Herstellung, diese enthaltende Heilmittel und die Verbindungen zur Verwendung als Heilmittel

Gegenstand der Erfindung sind Verbindungen der Formel I, die der Gruppe der 5-Sulfamoylorthanilsäuren zuzuordnen sind, sowie deren physiologisch verträgliche Salze.

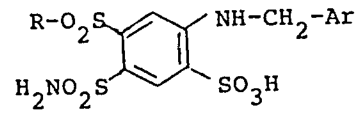

(I)

In der Formel bedeuten :

R Alkyl, Alkenyl, Cycloalkyl oder Cycloalkylalkyl mit je bis zu 10 C-Atomen oder Phenyl,

Ar Phenyl, Thienyl oder Furyl.

Soweit R Alkyl oder Alkenyl darstellt, kann es geradkettig oder verzweigt sein. Bevorzugt sind als Alkyle oder Alkylene diejenigen mit 4 bis 6 C-Atomen. Soweit R einen cyclischen Rest darstellt, sind Ringe mit 5 bis 8 Gliedern bevorzugt. Demgemäß kann als R insbesondere Cyclopentyl, Cyclohexyl, Cycloheptyl, Cyclohexylmethyl, n-Butyl, Isobutyl, n-Pentyl oder n-Hexyl stehen. Als Ar sind in erster Linie 2-Furyl und 2-Thienyl von Interesse.

Zur Salzbildung eignen sich alle physiologisch verträglichen Kationen, insbesondere Alkali- oder Erdalkaliionen, Ammonium oder substituiertes Ammonium.

Gegenstand der Erfindung ist ferner ein Verfahren zu Herstellung der Verbindungen der Formel I. Das Verfahren ist dadurch gekennzeichnet, daß man

a) einen Ester der Formel II

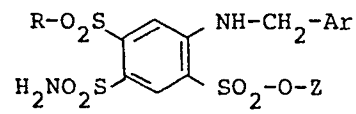

(II)

in der Z Aryl bedeutet, alkalisch verseift ;

b) eine Verbindung der Formel III

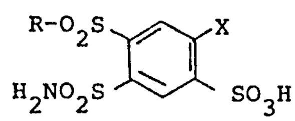

(III)

in der X Halogen bedeutet oder deren Salz mit einem Amin der Formel

$$H_2N—CH_2—Ar \qquad (IV)$$

umsetzt oder

c) eine Verbindung der Formel V

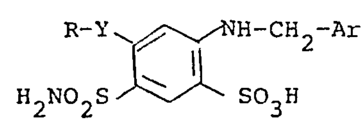

(V)

in der Y —S— oder —SO— bedeutet, oder deren Salz, zum Sulfon oxidiert und gegebenenfalls die erhaltene Verbindung in die freie Säure oder ein Salz überführt.

Bei der Verfahrensweise a) kommt als Rest Z im Ausgangsmaterial der Formel II prinzipiell jeder aromatische Rest infrage ; für die technische Synthese verwendet man jedoch mit besonderem Vorteil die einfach herzustellenden Phenyl- oder Kresylester. Die alkalische Verseifung der Ester wird vorzugsweise im wäßrigen Medium mit einer anorganischen Base, insbesondere mit überschüssiger 1- bis 5n Natronlauge oder Kalilauge, ausgeführt.

Beispielsweise können die Phenyl- oder Kresylester durch 2n wäßrige Natron- oder Kalilauge auf dem Dampfbad innerhalb einer Stunde vollständig verseift werden. Aus der klaren Verseifungslösung kristallisieren die Endprodukte in Form ihrer Natrium- bzw. Kaliumsalze sofort aus, wenn man die Lösung bei Raumtemperatur mit einer Mineralsäure, vorzugsweise Salzsäure, oder auch einer organischen Säure wie Essigsäure neutralisiert. Die Kaliumsalze sind generell schwerer wasserlöslich als die entsprechenden Natriumsalze. Das bei der Verseifung freiwerdende Phenol fällt teilweise zusammen mit dem Verfahrensprodukt aus, insbesondere dann, wenn an Stelle des Phenylesters ein Kresyl-, Xylyl-, Nitrophenyl-, Chlorphenyl- oder Naphthylester als Ausgangsmaterial verwendet wurde. Der Rest der

Phenole kann sodann durch gründliches Waschen der Fällung mit einem organischen Lösungsmittel abgetrennt werden. Hierzu dienen Lösungsmittel, in denen die Produkte praktisch unlöslich sind, wie Äthanol, Isopropanol, Diäthyläther, Diisopropyläther, Aceton oder Tetrahydrofuran. Eventuell dann noch vorhandene letzte Spuren Phenol können durch nachfolgendes Umkristallisieren der Produkte aus wäßrigen Alkoholen entfernt werden.

Die Ausgangsstoffe der Formel II können beispielsweise aus den in der deutschen Auslegeschrift 2 718 871 beschriebenen 2,4-Dichlor-5-sulfamoylbenzolsulfosäurearylestern der Formel VI gemäß dem folgenden Schema dargestellt werden :

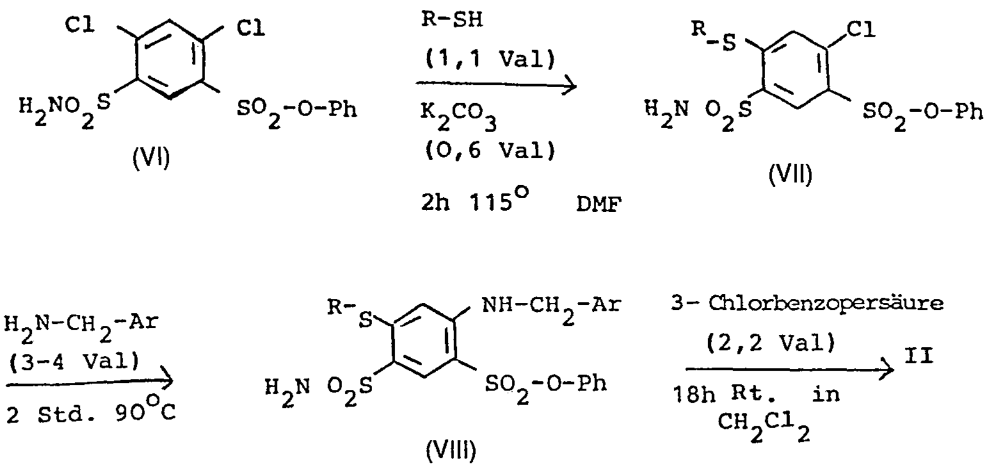

(VI) (VII) (VIII)

Bei der Verfahrensweise (b) werden als Ausgangsstoffe der Formel III vorzugsweise die Alkalisalze von Verbindungen eingesetzt, in denen X Fluor oder Chlor bedeutet. Die Umsetzung mit dem Amin der Formel IV erfolgt, falls X Fluor bedeutet, vorteilhaft bei einer Temperatur zwischen 80 und 100 °C und, falls X Chlor bedeutet, vorteilhaft bei einer Temperatur zwischen 120 und 140 °C. Zur Bindung des entstehenden Halogenwasserstoffs kann man dem Reaktionsansatz einen Säurebinder, z. B. Natriumcarbonat, Kaliumcarbonat oder ein tertiäres Amin zusetzen. Besonders vorteilhaft verwendet man die Base IV selbst als Säurebinder indem man sie in 2- bis 3-molarem Überschuß einsetzt.

Als Lösungsmittel bei dieser Reaktion dienen insbesondere stark polare mit Wasser mischbare organische Lösungen wie Dimethylsulfoxid oder Dimethylformamid.

Nach beendeter Umsetzung werden Lösungsmittel und überschüssiges Amin abgetrennt, vorteilhaft durch vollständiges Abdestillieren. Der Rückstand wird mit Wasser behandelt und das rohe Endprodukt wie bei der Verfahrensweise (a) beschrieben gereinigt. Die Ausgangsstoffe der Formel III können in einfacher Weise durch Oxidation der Verbindungen VII (mit $H_2O_2$ oder einer organischen Persäure) und nachfolgende alkalische Verseifung erhalten werden. Bei der Verfahrensweise (c) wird ein Thioäther bzw. ein Sulfoxid der Formel V zum entsprechenden Sulfon oxidiert. Falls Ar Phenyl oder Thienyl bedeutet, führt man diese Oxidation vorteilhaft mit Perhydrol in Eisessig oder mit Peressigsäure/Eisessig bei einer Temperatur zwischen 20 und 60 °C aus. Verbindungen der Formel V, in denen Ar Furyl bedeutet, werden vorteilhaft in verdünnter Lösung bei Raumtemperatur mit der berechneten Menge Persäure oxidiert. Als Lösungsmittel kann hierbei beispielsweise eine Mischung von Methylenchlorid und Dimethylformamid und als Oxidationsmittel 3-Chlorperbenzoesäure infrage kommen. Zur Isolierung der Endprodukte wird nach Abtrennen des Lösungsmittels vorteilhaft aus wäßriger 1n $KHCO_3$- oder 1n $NaHCO_3$-Lösung umkristallisiert.

Die Ausgangsstoffe der Formel V können in einfacher Weise durch Verseifung oder auch durch Oxidation zum Sulfoxid mit nachfolgender Verseifung aus Sulfosäurearylestern der Formel VIII erhalten werden.

Die erfindungsgemäßen Verbindungen sind in Form ihrer Alkali-, Erdalkali- oder Ammoniumsalze stabile, farblose und gut kristallisierende Stoffe. In Wasser sind sie bei Raumtemperatur nicht besonders gut löslich. Deshalb lassen sie sich aus Wasser oder Wasser mit organischen Lösungsmitteln wie Alkoholen, Aceton, Dioxan, Tetrahydrofuran oder Dimethylformamid ausgezeichnet umkristallisieren. Von den Metallsalzen sind die Natriumsalze am besten wasserlöslich. Sie lassen sich daher besonders einfach in wässriger Lösung durch doppelte Umsetzung mit entsprechenden wasserlöslichen Salzen anderer Metalle in andere Metallsalze überführen. Noch besser wasserlöslich als die Natriumsalze sind die Salze mit Hydroxyaminen, beispielsweise mit Mono-, Di- oder Triäthanolamin oder mit Glucosamin.

Die Verbindungen können auch als freie Sulfosäuren isoliert werden. Beispielsweise gelingt dies durch die Behandlung der Natriumsalze mit 5n HCl. Die freien Säuren sind jedoch nicht sehr stabil, insbesondere dann nicht, wenn Ar Furyl darstellt. Für therapeutische Zwecke kommen die freien Säuren daher weniger infrage. Für Arzneimittelzubereitungen besonders geeignet sind hingegen die Natrium- und Kaliumsalze. Große pharmakologische Bedeutung haben auch die Salze mit basischen kaliumretinierenden Verbindungen, wie Amilorid oder Triamteren oder auch die Salze mit basischen Antihypertensiva

wie Clonidin, Dihydralazin oder Guanethidin oder mit β-Blockern wie Propranolol, Timolol, Penbutolol oder Pindolol. Die erfindungsgemäßen Verbindungen sind Salidiuretika vom Furosemid-Typ mit außerordentlich hoher Potenz. Gegenüber bekannten Verbindungen dieses Typs zeichnen sie sich ferner durch eine besonders niedrige Kaliumausscheidung und eine ausgeprägte uricosurische Wirkungskomponente aus.

Für die Humantherapie kommt vor allem die orale Applikation der Produkte, vorzugsweise in Form von Tabletten, Dragees oder Kapseln mit 0,1 bis 50 mg Wirkstoff in Frage, daneben auch die i. v. Applikation mit wässrigen Injektionslösungen mit 0,1 bis 10 mg Wirkstoff.

Beispiel 1

N-(2-Furylmethyl)-4-cyclohexylsulfonyl-5-sulfamoylorthanilsäure-natrium

55,5 g N-(2-Furylmethyl)-4-cyclohexylsulfonyl-5-sulfamoylorthanilsäurephenylester (0,1 Mol) vom Schmp. 133 °C (aus Methanol umkristallisiert) werden mit 0,4 l 2n NaOH 1 Stunde bei 95 °C gerührt. Nachfolgend wird die klare Reaktionslösung auf Raumtemperatur abgekühlt und mit 5n HCl auf pH 7 eingestellt. Nach einstündigem Stehen bei Raumtemperatur saugt man das kristallin abgeschiedene Endprodukt ab, wäscht nacheinander mit Eiswasser und abs. Äthanol und trocknet bei 90 °C.

Ausbeute : 44 g (88 % d. Th.), Zers.-P. 300 °C

Beispiel 2

N-(2-Furylmethyl)-4-cyclohexylsulfonyl-5-sulfamoylorthanilsäure-kalium

Die Lösung von 48,5 g N-(2-Furylmethyl)-4-cyclohexylmercapto-5-sulfamoylorthanilsäure-kalium (0,1 Mol) vom Zers.-P. 227 °C (aus Wasser umkristallisiert) in 1,0 l Dimethylformamid versetzt man bei Raumtemperatur portionsweise und unter Rühren mit 42 g 90-prozentiger 3-Chlorperbenzoesäure (0,22 Mol). Nach Stehen über Nacht bei Raumtemperatur zieht man das Dimethylformamid im Vakuum ab, versetzt den Rückstand mit 1,0 l Wasser und stellt mit 2n KOH pH 8 ein. Nach einstündigem Stehen bei 15° wird das kirstallin abgeschiedene Endprodukt abgesaugt und durch Umkristallisieren aus Wasser, unter Zusatz von Aktivkohle, gereinigt.

Ausbeute : 41 g (79 % d. Th.), Zers.-P. 305 °C.

Beispiel 3

N-(2-Thienylmethyl)-4-cyclohexylsulfonyl-5-sulfamoylorthanilsäure-natrium

57,1 g N-(2-Thienylmethyl-4-cyclohexylsulfonyl-5-sulfamoylorthanilsäurephenylester (0,1 Mol) vom Schmp. 193 °C werden analog Beispiel 1 mit 2n NaOH verseift und das Endprodukt wie dort beschrieben isoliert.

Ausbeute : 48,5 g (84 % d. Th.), Zers.-P. 310 °C.

Beispiel 4

N-Benzyl-4-cyclohexylsulfonyl-5-sulfamoylorthanilsäure-natrium

56,4 g N-Benzyl-4-cyclohexylsulfonyl-5-sulfamoylorthanilsäurephenylester (0,1 Mol) werden analog Beispiel 1 mit 0,4 l 2n NaOH verseift und das Endprodukt aus 30-proz. Äthanol umkristallisiert.

Ausbeute : 43 g (84 % d. Th.), Zers.-P. 296 °C

Beispiel 5

N-(2-Furylmethyl)-4-phenylsulfonyl-5-sulfamoylorthanilsäure-kalium

45,0 g 2-Chlor-4-phenylsulfonyl-5-sulfamoylbenzolsulfosäurekalium (0,1 Mol) werden mit einer Mischung von 0,1 l Furfurylamin und 0,1 l Dimethylsulfoxid 2 Stunden bei 125-130 °C gerührt. Nachfolgend destilliert man überschüssiges Furfurylamin und Dimethylsulfoxid im Vakuum ab, versetzt den Rückstand mit 0,1 l Wasser und stellt die Mischung mit 5n HCl auf pH 7 ein. Nach einstündigem Stehen bei Raumtemperatur wird das kristallin ausgefallene Endprodukt abgesaugt und durch Umkristallisieren aus Wasser gereinigt.

Ausbeute : 35 g (70 % d. Th.), Zers.-P. 270 °C.

Beispiel 6

N-(2-Furylmethyl)-4-cycloheptylsulfonyl-5-sulfamoylorthanilsäure-natrium

56,9 g N-(2-Furylmethyl)-4-cycloheptylsulfonyl-5-sulfamoylorthanilsäurephenylester (0,1 Mol) vom Schmp. 163 °C (aus Methanol umkristallisiert) werden analog Beispiel 1 mit Natronlauge verseift und das Endprodukt wie dort beschrieben isoliert.

Ausbeute : 44 g (86 % d. Th.), Zers.-P. 284 °C.

Beispiel 7

N-(2-Furylmethyl)-4-n-butylsulfonyl-5-sulfamoylorthanilsäure-kalium

45,8 g N-(2-Furylmethyl)-4-n-butylmercapto-5-sulfamoylorthanilsäure-kalium (0,1 Mol) vom Zers.-P. 245 °C (aus Wasser umkristallisiert) werden in 0,1 l Dimethylsulfoxid gelöst, die Lösung mit 0,3 l Methylenchlorid verdünnt, bei Raumtemperatur unter Rühren mit 48 g 90-proz. 3-Chlorperbenzoesäure (0,25 Mol) versetzt und über Nacht bei Raumtemperatur belassen. Man zieht dann das Lösungsmittel im Vakuum ab, digeriert den Rückstand zur Entfernung der 3-Chlorbenzoesäure mit 0,3 l warmer 1n $KHCO_3$ Lösung und reinigt das ungelöst bleibende Endprodukt durch nachfolgendes Umkristallisieren aus Wasser.

Ausbeute : 24 g (49 d. Th.), Zers.-P. 330 °C.

Beispiel 8

N-(2-Furylmethyl)-4-n-propylsulfonyl-5-sulfamoylorthanilsäurephenylester (0,1 Mol) vom Schmp. 145 °C (aus Methanol) umkristallisiert) werden analog Beispiel 1 mit NaOH verseift und das Endprodukt wie dort beschrieben isoliert.

Ausbeute : 35 g (76 % d. Th.), Zers.-P. 320 °C.

Beispiel 9

N-(2-Furylmethyl)-4-cyclopentylsulfonyl-5-sulfamoylorthanilsäure-natrium

54,1 g N-(2-Furylmethyl)-4-cyclopentylsulfonyl-5-sulfamoylorthanilsäurephenylester (0,1 Mol) vom Schmp. 169 °C (umkristallisiert aus einer Mischung Methanol/Dimethylformamid 1 : 2) werden analog Beispiel 1 mit NaOH verseift. Das mit HCl bei pH 7 ausgefällte Endprodukt wird auf der Nutsche mit Wasser und nachfolgend mit Äthanol gewaschen und bei 90 °C getrocknet.

Ausbeute : 40 g (82 % d. Th.), Zers.-P. 285 °C.

Beispiel 10

N-(2-Furylmethyl)-4-allylsulfonyl-5-sulfamoylorthanilsäure-kalium

51,3 g N-(2-Furylmethyl)-4-allylsulfonyl-5-sulfamoylorthanilsäurephenylester (0,1 Mol) vom Schmp. 138 °C werden mit 0,4 l 2n KOH eine halbe Stunde unter Rühren auf dem Dampfbad erwärmt. Nach Abkühlen der Reaktionslösung auf Raumtemperatur und Neutralisation mit 5n HCl wird zur Trockne eingedampft, der Rückstand mit 0,1 l heißem Dimethylformamid extrahiert und das Endprodukt aus der filtrierten Lösung durch Zugabe von 0,4 l Diisopropyläther kristallin gefällt.

Ausbeute : 35 g (74 % d. Th.), Zers.-P. 125 °C.

Beispiel 11

N-(2-Furylmethyl)-4-cyclohexylmethylsulfonyl-5-sulfamoylorthanilsäure-natrium

56,7 g N-(2-Furylmethyl)-4-cyclohexylmethylsulfonyl-5-sulfamoylorthanilsäurephenylester (0,1 Mol) vom Schmp. 152 °C (aus Äthanol) werden mit 0,4 l 2n NaOH 45 Minuten unter Rühren auf dem Dampfbad erwärmt und das Endprodukt analog Beispiel 1 isoliert.

Ausbeute : 46 g (90 % d. Th.), Zers.-P. 350 °C.

Beispiel 12

N-(2-Furylmethyl)-4-methylsulfonyl-5-sulfamoylorthanilsäure-kalium

48,6 g N-(2-Furylmethyl)-4-methylsulfonyl-5-sulfamoylorthanilsäurephenylester (0,1 Mol) vom Schmp. 175 °C (aus Methanol) werden durch einstündiges Erwärmen mit 0,4 l 2n KOH auf dem Dampfbad verseift und das Endprodukt analog Beispiel 1 isoliert.

Ausbeute : 36,5 g (81 % d. Th.), Zers.-P. 225 °C.

Beispiel 13

N-(2-Thienylmethyl)-4-cyclopentylsulfonyl-5-sulfamoylorthanilsäure-natrium

55,7 g N-(2-Thienylmethyl)-4-cyclopentylsulfonyl-5-sulfamoylorthanilsäurephenylester (0,1 Mol) vom Schmp. 194 °C (aus Methanol) werden durch einstündiges Erhitzen mit 0,4 l 2n NaOH auf dem Dampfbad verseift und das Endprodukt analog Beispiel 1 isoliert.

Ausbeute : 42,5 g (85 % d. Th.), Zers.-P. 278 °C.

Beispiel 14

N-(2-Furylmethyl)-4-n-hexylsulfonyl-5-sulfamoylorthanilsäure-natrium

48,7 g N-(2-Furylmethyl)-4-n-hexylsulfonyl-5-sulfamoylorthanilsäurephenylester (0;1 Mol) vom Schmp. 140 °C (aus Methanol) werden mit 0,4 l 2n NaOH 45 Minuten rückfließend erhitzt. Nachfolgend stellt man die klare Reaktionslösung bei Raumtemperatur mit 5n HCl auf pH 7 ein, saugt nach einer Stunde das kristallin ausgefallene Endprodukt ab, wäscht mit absolutem Äthanol und trocknet bei 90 °C.

Ausbeute : 43 g (85 % d. Th.), Zers.-P. 209 °C.

Außer den vorstehend beschriebenen Verbindungen können in analoger Weise auch die folgenden Verfahrensprodukte erhalten werden :

N-(2-Furylmethyl)-4-äthylsulfonyl-5-sulfamoylorthanilsäure-kalium, N-(2-Thienylmethyl)-4-äthylsulfonyl-5-sulfamoylorthanilsäure-natrium, N-(2-Furylmethyl)-4-isopropylsulfonyl-5-sulfamoylorthanilsäure-natrium, N-(2-Thienylmethyl-4-n-propylsulfonyl-5-sulfamoylorthanilsäure-natrium, N-(2-Thienylmethyl)-4-n-butylsulfonyl-5-sulfamoylorthanilsäure-kalium, N-Benzyl-4-n-butylsulfonyl-5-sulfamoylorthanilsäure-natrium, N-(2-Furylmethyl-4-isobutylsulfonyl-5-sulfamoylorthanilsäure-kalium, N-(2-Thienylmethyl)-4-isobutylsulfonyl-5-sulfamoylorthanilsäure-kalium, N-(2-Thienylmethyl)-4-allylsulfonyl-5-sulfamoylorthanilsäure-natrium, N-(2-Furylmethyl)-4-n-pentylsulfonyl-5-sulfamoylorthanilsäure-kalium, N-(2-Furylmethyl)-4-n-octylsulfonyl-5-sulfamoylorthanilsäure-kalium, N-(2-Furylmethyl)-4-cyclopropylsulfonyl-5-sulfamoylorthanilsäure-kalium, N-(2-Furylmethyl)-4-cyclopropylmethylsulfonylorthanilsäure-natrium, N-(2-Furylmethyl)-4-cyclobutylmethylsulfonyl-5-sulfamoylorthanilsäure-natrium, N-(2-Furylmethyl)-4-cyclopentylmethylsulfonyl-5-sulfamoylorthanilsäure-kalium, N-(3-Furylmethyl)-4-cyclohexylsulfonyl-5-sulfamoylorthanilsäure-natrium, N-(2-Thienylmethyl)-4-cycloheptylsulfonyl-5-sulfamoylorthanilsäure-natrium, N-(2-Furylmethyl)-4-cyclooctylsulfonyl-5-sulfamoylorthanilsäure-kalium und N-(2-Furylmethyl)-4-cyclooctylmethylsulfonyl-5-sulfamoylorthanilsäure-natrium.

**Ansprüche** (für die Vertragsstaaten : BE, CH, DE, FR, GB, IT, LI, NL, SE)

1. Verbindungen der Formel I

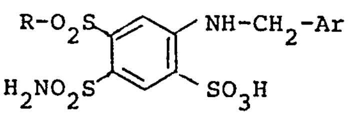

(I)

in der bedeuten

R Alkyl, Alkenyl, Cycloalkyl oder Cycloalkylalkyl mit je bis zu 10 C-Atomen oder Phenyl

Ar Phenyl, Thienyl oder Furyl

sowie deren physiologisch verträgliche Salze.

2. Verbindungen gemäß Anspruch 1, worin

R Cyclopentyl, Cyclohexyl, Cycloheptyl, Cyclohexylmethyl, n-Butyl, Isobutyl, n-Pentyl oder n-Hexyl und

Ar 2-Furyl oder 2-Thienyl ist.

3. Verbindung gemäß Anspruch 1, worin R n-Butyl und Ar 2-Furyl ist.

4. Verbindung gemäß Anspruch 1, worin R Cyclohexyl und Ar 2-Furyl ist.

5. Verfahren zur Herstellung einer Verbindung gemäß Anspruch 1, dadurch gekennzeichnet, daß man

a) einen Ester der Formel II,

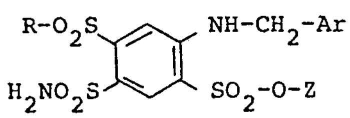

(II)

in der Z Aryl bedeutet, alkalisch verseift ;

b) eine Verbindung der Formel III,

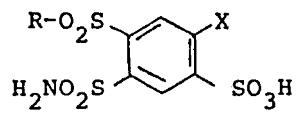

(III)

in der X Halogen bedeutet oder deren Salz mit einem Amin der Formel IV

$$H_2N—CH_2—Ar \qquad (IV)$$

umsetzt oder

c) eine Verbindung der Formel V,

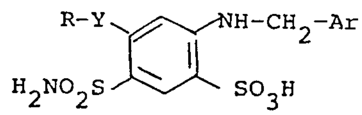

(V)

in der Y —S— oder —SO— bedeutet, oder deren Salz, zum Sulfon oxidiert und gegebenenfalls die erhaltene Verbindung in die freie Säure oder ein Salz überführt.

6. Heilmittel, enthaltend eine Verbindung gemäß einem der Ansprüche 1-4.

7. Verbindung gemäß einem der Ansprüche 1-4 zur Verwendung als Heilmittel.

**Ansprüche** (für den Vertragsstaat AT)

1. Verfahren zur Herstellung einer Verbindung der Formel I

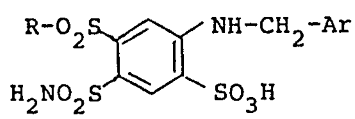

(I)

in der bedeuten

R Alkyl, Alkenyl, Cycloalkyl oder Cycloalkylalkyl mit je bis zu 10 C-Atomen oder Phenyl

Ar Phenyl, Thienyl oder Furyl

sowie von deren physiologisch verträglichen Salzen, dadurch gekennzeichnet, daß man

a) einen Ester der Formel II,

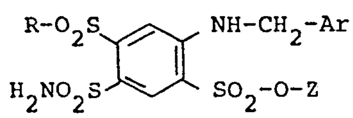

(II)

in der Z Aryl bedeutet, alkalisch verseift ;

b) eine Verbindung der Formel III,

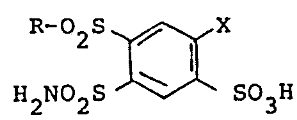

(III)

in der X Halogen bedeutet oder deren Salz mit einem Amin der Formel IV

$$H_2N—CH_2—Ar \qquad (IV)$$

umsetzt oder

c) eine Verbindung der Formel V,

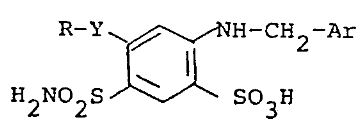

(V)

in der Y —S— oder —SO— bedeutet, oder deren Salz, zum Sulfon oxidiert und gegebenenfalls die erhaltene Verbindung in die freie Säure oder ein Salz überführt.

2. Verfahren gemäß Anspruch 1, dadurch gekennzeichnet, daß man eine Verbindung der Formel I worin

R Cyclopentyl, Cyclohexyl, Cycloheptyl, Cyclohexylmethyl, n-Butyl, Isobutyl, n-Pentyl oder n-Hexyl und

Ar 2-Furyl oder 2-Thienyl ist, herstellt.

3. Verfahren gemäß Anspruch 1, dadurch gekennzeichnet, daß man eine Verbindung der Formel I worin R n-Butyl und Ar 2-Furyl ist, herstellt.

4. Verfahren gemäß Anspruch 1, dadurch gekennzeichnet, daß man eine Verbindung der Formel I worin R Cyclohexyl und Ar 2-Furyl ist, herstellt.

**Claims** (for the Contracting States : BE, CH, DE, FR, GB, IT, LI, NL, SE)

1. Compounds of the formula I

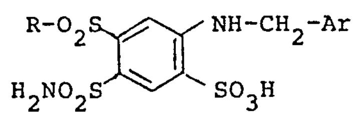

(I)

in which

R denotes alkyl, alkenyl, cycloalkyl or cycloalkylalkyl, each of which has up to 10 C atoms or phenyl, and

Ar denotes phenyl, thienyl or furyl

and physiologically acceptable salts thereof.

2. Compounds according to Claim 1, wherein

R is cyclopentyl, cyclohexyl, cycloheptyl, cyclohexylmethyl, n-butyl, isobutyl, n-pentyl or n-hexyl and Ar is 2-furyl or 2-thienyl.

3. Compound according to Claim 1, wherein R is n-butyl and Ar is 2-furyl.

4. Compound according to Claim 1, wherein R is cyclohexyl and Ar is 2-furyl.

5. Process for the preparation of a compound according to Claim 1, which comprises

a) saponifying under alkaline conditions an ester of the formula II

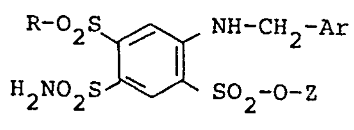

(II)

in which Z denotes aryl ;

b) reacting a compound of the formula III

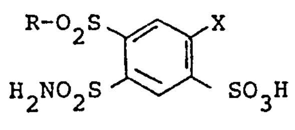

(III)

in which X denotes halogen, or a salt thereof, with an amine of the formula IV

$$H_2N—CH_2—Ar \quad (IV)$$

or

c) oxidizing a compound of the formula V

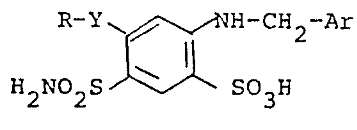

(V)

in which Y denotes —S— or —SO—, or a salt therof, to give the sulfone and, if appropriate, converting the resulting compound into the free acid or into a salt.

6. Medicament, containing a compound according to any one of claims 1-4.

7. Compound according to any one of claims 1-4 for the use as medicament.

**Claims** (for the Contracting State AT)

1. Process for the preparation of a compound of formula I

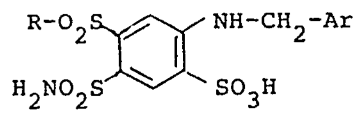

(I)

in which

R denotes alkyl, alkenyl, cycloalkyl or cycloalkylalkyl, each of which has up to 10 C atoms, or phenyl and

Ar denotes phenyl, thienyl or furyl,

and also physiologically acceptable salts thereof, which comprises

a) saponifying under alkaline conditions an ester of the formula II

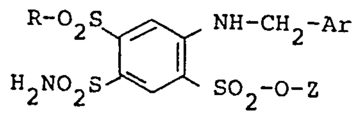

(II)

in which Z denotes aryl ;

b) reacting a compound of the formula III

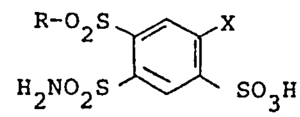

(III)

in which X denotes halogen, or a salt thereof, with an amine of the formula IV

$$H_2N—CH_2—Ar \quad (IV)$$

or

c) oxidizing a compound of the formula V

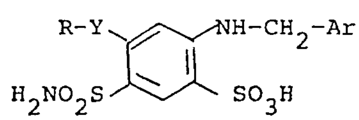

(V)

in which Y denotes —S— or —SO—, or a salt thereof, to give the sulfone and, if appropriate, converting the resulting compound into the free acid or into a salt.

2. Process according to claim 1, characterized in that a compound of formula I is prepared in which R is cyclopentyl, cyclohexyl, cycloheptyl, cyclohexylmethyl, n-butyl, isobutyl, n-pentyl or n-hexyl and Ar is 2-furyl or 2-thienyl.

3. Process according to claim 1, characterized in that a compound of formula I is prepared in which R is n-butyl and Ar is 2-furyl.

4. Process according to claim 1, characterized in that a compound of formula I is prepared in which R is cyclohexyl and Ar is 2-furyl.

**Revendications** (pour les Etats contractants : BE, CH, DE, FR, GB, IT, LI, NL, SE)

1. Composés répondant à la formule I

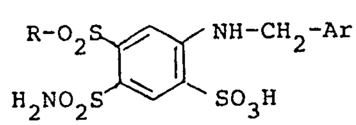

(I)

dans laquelle

R représente un groupe alcoyle, alcényle, cycloalcoyle ou cycloalcoylalcoyle ayant jusqu'à 10 atomes de carbone chacun ou un groupe phényle,

Ar représente un groupe phényle, thiényle ou furyle, et leurs sels physiologiquement acceptables.

2. Composés selon la revendication 1, dans lesquels

R représente un groupe cyclopentyle, cyclohexyle, cycloheptyle, cyclohexylméthyle, n-butyle, isobutyle, n-pentyle ou n-hexyle et

Ar représente un groupe 2-furyle ou 2-thiényle.

3. Composé selon la revendication 1, dans lequel R est le groupe n-butyle et Ar le groupe 2-furyle.

4. Composé selon la revendication 1, dans lequel R est le groupe cyclohexyle et Ar le groupe 2-furyle.

5. Procédé pour la préparation d'un composé selon la revendication 1, caractérisé en ce que :

a) on saponifie dans des conditions alcalines un ester de formule II

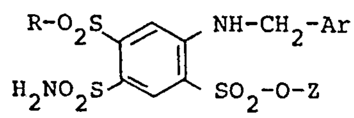
(II)

dans laquelle Z est un groupe aryle ;

b) on fait réagir un composé de formule III

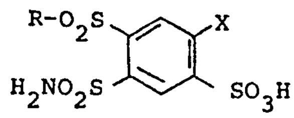
(III)

dans laquelle X représente un halogène, ou un de ses sels, avec une amine de formule IV

$$H_2N—CH_2—Ar \quad (IV)$$

ou

c) on oxyde en sulfone un composé de formule V

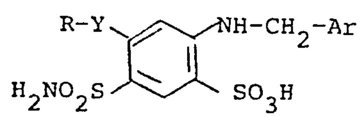
(V)

dans laquelle Y représente —S— ou —SO—, ou un de ses sels ; et éventuellement on convertit le composé obtenu en l'acide libre ou en un sel.

6. Médicament contenant un composé selon l'une quelconque des revendications 1 à 4.

7. Composé selon l'une quelconque des revendications 1 à 4 destiné à être utilisé comme médicament.

**Revendications** (pour l'Etat contractant AT)

1. Procédé pour la préparation d'un composé de formule I

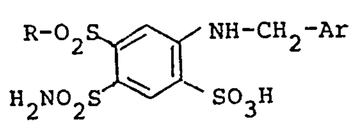
(I)

dans laquelle

R représente un groupe alcoyle, alcényle, cycloalcoyle ou cycloalcoylalcoyle, ayant jusqu'à 10 atomes de carbone chacun, ou un groupe phényle,

Ar représente un groupe phényle, thiényle ou furyle,

ainsi que leurs sels physiologiquement acceptables, caractérisé en ce que

a) on saponifie dans des conditions alcalines un ester de formule II

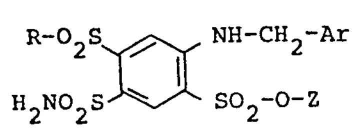
(II)

dans laquelle Z représente un groupe aryle,

b) on fait réagir un composé de formule III

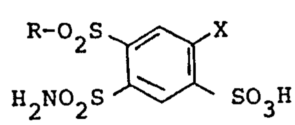
(III)

dans laquelle X est un halogène, ou un de ses sels, avec une amine de formule IV

$$H_2N—CH_2—Ar \qquad (IV)$$

ou

c) on oxyde en sulfone un composé de formule V

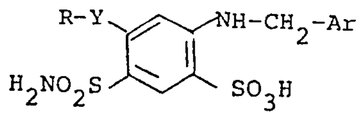

(V)

dans laquelle Y représente —S— ou —SO—, ou un de ses sels, et éventuellement on convertit le composé obtenu en l'acide libre ou en un sel.

2. Procédé selon la revendication 1, caractérisé en ce qu'on prépare un composé de formule I, dans lequel

R représente un groupe cyclopentyle, cyclohexyle, cycloheptyle, cyclohexylméthyle, n-butyle, isobutyle, n-pentyle ou n-hexyle et

Ar représente un groupe 2-furyle ou 2-thiényle.

3. Procédé selon la revendication 1, caractérisé en ce qu'on prépare un composé de formule I, dans lequel R est le groupe n-butyle et Ar est le groupe 2-furyle.

4. Procédé selon la revendication 1, caractérisé en ce qu'on prépare un composé de formule I, dans lequel R est le groupe cyclohexyle et Ar est le groupe 2-furyle.